# EUROPEAN PATENT APPLICATION

(11) **EP 1 764 685 A2**
(43) Date of publication of application: **21.03.2007**
(21) Application number: 06002274.6
(22) Date of filing: 03.02.2006
(51) Int. Cl.: G06F 9/44

(54) **Method for displaying two-dimensional representation relating to an object and corresponding apparatus and program storage medium**

(30) Priority: 14.09.2005 JP 2005267667
(71) Applicant: Fujitsu Ltd., Kawasaki-shi, Kanagawa 211-8588 (JP)
(72) Inventor: Yokoyama, Masaru c/o FUJITSU KYUSHU SYS. ENG. LTD, Fukuoka-shi Fukuoka 814-8589 (JP)
(74) Representative: Sunderland, James Harry

(57) **Abstract**

A display program for allowing a user to easily understand changes of observed values is provided. Information necessary for drawing is read from storage means to generate drawing data from the read information, and a bound graph, which is a two-dimensional representation, is displayed as an animation in which measurement points of nodes move up and down over times. This allows the user to observe variations of observed values by using the movement of the measurement points in addition to coloring, which is easy-to-analyze for the user.

## Description

### BACKGROUND OF THE INVENTION

### 1. Field of the Invention

The present invention relates to a program for displaying observed data and network information in an easy-to-analyze manner.

### 2. Description of the Related Art

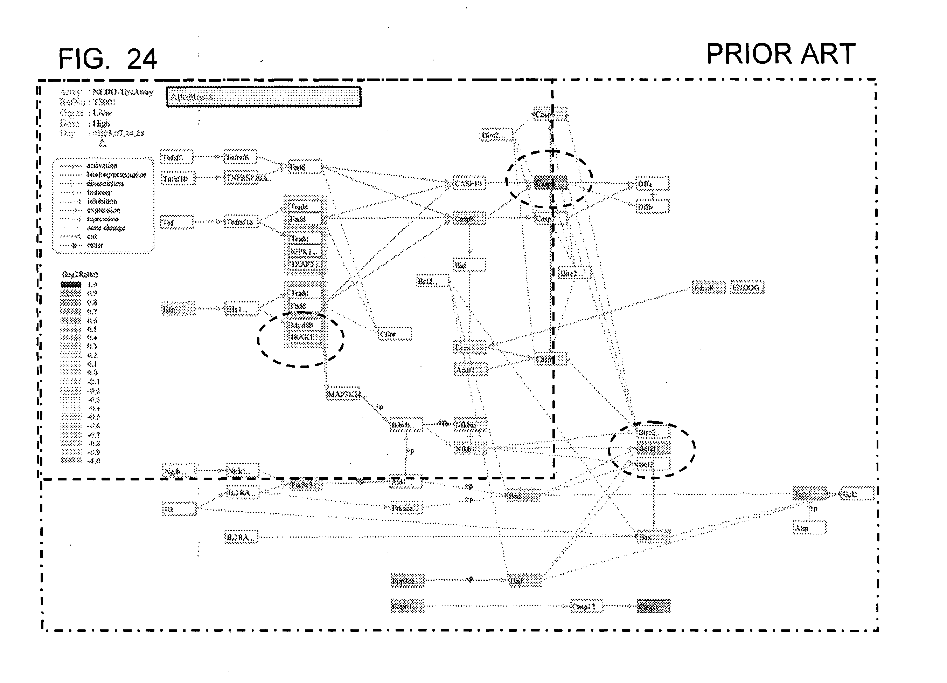

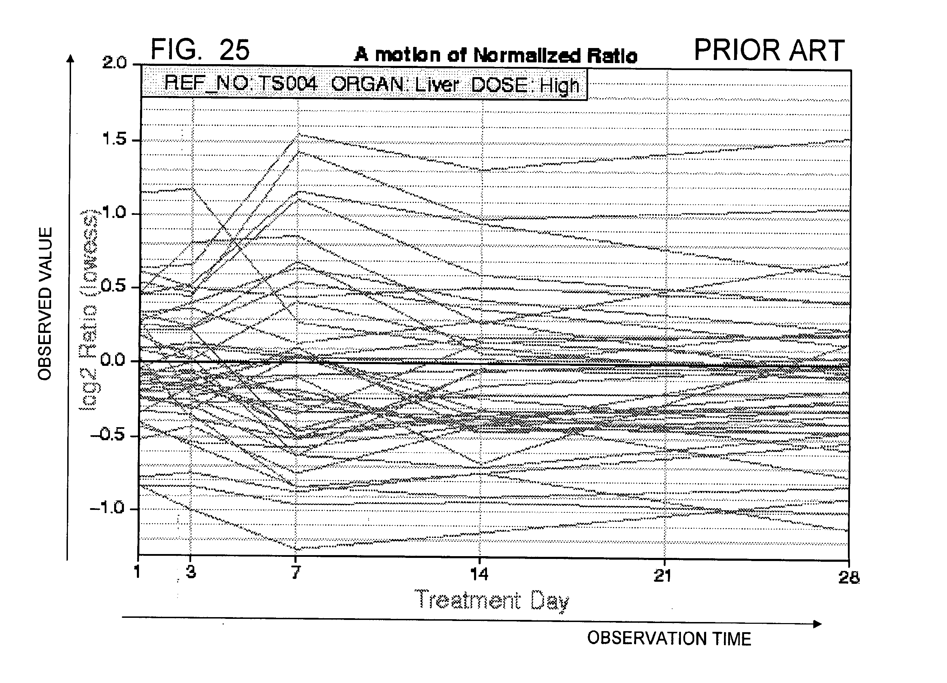

In the related art, there is a device adapted to display observed data on a pathway diagram. Fig. 24 is a pathway diagram according to the related art. The device displays nodes on the pathway diagram in different colors from one observation time to another depending on the expression ratio. Along with the change of observation times, the nodes change in color. A user performs analysis while observing the changes in color and taking a genetic correlation displayed on the pathway diagram into consideration. In addition to the pathway diagram, the device also displays the observed data on a line graph with expression ratios in the y-axis and observation times in the x-axis. Fig. 25 is a line graph showing observed values according to the related art. While observing the colors of the nodes and the genetic correlation on the pathway diagram, the user reviews the line graph, as needed.

### SUMMARY OF THE INVENTION

The device of the related art with the above-described arrangement allows the user to perform analysis using the pathway diagram and the line graph of the observed data. However, the device has a problem in that the pathway diagram showing a correlation between genes has many blanks so that it is difficult to fully display the pathway diagram in a display region of the device. In Fig. 24, a region defined by a dotted rectangular line corresponds to the display region, and a region defined by a one-dot-chain rectangular line represents the full size of the pathway diagram. The entire pathway diagram is not included in the display region. Since all nodes surrounded by dotted elliptic lines are not displayed in the display region, it is difficult to understand control relationships.

If the desired pathway diagram is included in the display region, it is difficult to draw attention to both nodes located at an end and the opposite end of the display region. In a case where such attention is needed, a large load is placed on the user, and oversights may often occur during the analysis.

Although the device of the related art allows the user to review the line graph of the observed data together with the pathway diagram to thereby implement an appropriate analysis, there arises another problem. That is, the displayed line graph causes the pathway diagram to be hidden, and the user needs to switch between the views, which is time-consuming for the user. Moreover, the hidden view relies on unreliable human memory, thus preventing a high-accuracy analysis. For example, in order to refer to the exact observed values of the nodes surrounded by the dotted elliptic lines illustrated in Fig. 24, it is necessary to switch the view to the line graph illustrated in Fig. 25 to specify lines defined by dotted circular lines in the line graph. By displaying the line graph illustrated in Fig. 25, the pathway diagram illustrated in Fig. 24 is completely hidden, and the control relationships resort to only the memory of the user. In such a case, it is difficult to analyze a considerable change affecting the nodes, which accounts for small changes. This is of the particular importance for some targets for analysis.

In order to overcome the foregoing problems, it is an object of the present invention to provide a display program for allowing a user to more easily understand changes of observed values.

According to an aspect of the present invention, a program storage medium storing a display program is provided. The display program allows a computer to execute a reading step of reading observation information of an object from a storage means, a generating step of generating drawing-data of the object on the basis of an observation time and an observed value, and a displaying step of displaying two-dimensional representations corresponding to a plurality of the observation times in turn on a display device. The observation information includes the observation time and the observed value. The object has been observed at the observation time and is associated with the observed value. The observation time and the observed value has been stored in the storage means beforehand. The two-dimensional representation is generated on the basis of the drawing-data. The two-dimensional representation has a first coordinate axis for representing an identifier of the object and a second coordinate axis for representing the observed value of the object.

According to another aspect of the present invention, a display apparatus is provided. The display apparatus includes a reading means for reading observation information of an object from a storage means, a generating means for generating drawing-data of the object on the basis of an observation time and an observed value; and a displaying means for displaying two-dimensional representations corresponding to a plurality of the observation times in turn on a display device. The observation information includes the observation time and the observed value. The object has been observed at the observation time and is associated with the observed value. The observation time and the observed value has been stored in the storage means beforehand. The two-dimensional representation is generated on the basis of the drawing-data. The two-dimensional representation has a first coordinate axis for representing an identifier of the object and a second coordinate axis for representing the observed value of the object.

According to another aspect of the present invention, a display method is provided. The display method includes a reading step of reading observation information of an object from a storage means, a generating step of generating drawing-data of the object on the basis of an observation time and an observed value, and a displaying step of displaying two-dimensional representations corresponding to a plurality of the observation times in turn on a display device. The observation information includes the observation time and the observed value. The object has been observed at the observation time and is associated with the observed value. The observation time and the observed value has been stored in the storage means beforehand. The two-dimensional representation is generated on the basis of the drawing-data. The two-dimensional representation has a first coordinate axis for representing an identifier of the object and a second coordinate axis for representing the observed value of the object.

### BRIEF DESCRIPTION OF THE DRAWINGS

Fig. 1 is a schematic diagram illustrating a display program according to an embodiment of the present invention;
Fig. 2 is a configuration diagram of a system used in a display program according to an embodiment of the present invention;
Fig. 3 is an example of a hardware configuration according to an embodiment of the present invention;
Fig. 4 is a diagram illustrating an example bound graph in a normal view mode according to an embodiment of the present invention;
Figs. 5A to 5C are diagrams illustrating an example bound graph in a vector view mode according to an embodiment of the present invention;
Figs. 6A to 6C are diagrams illustrating an example bound graph in a view mode based on the movement distance from the initial observation time according to an embodiment of the present invention;
Figs. 7A to 7D are diagrams illustrating an example bound graph in a view mode based on the distance between two or more parameters according to an embodiment of the present invention;
Fig. 8 is a diagram specifically illustrating the view mode based on the distance between two or more parameters according to an embodiment of the present invention;
Fig. 9 is a diagram specifically illustrating the view mode based on the distance between two or more parameters according to an embodiment of the present invention;
Fig. 10 is a diagram illustrating a bound graph in a data-type-based color-coded parameter view mode according to an embodiment of the present invention;
Fig. 11 is a diagram illustrating a demonstration of a control relationship on a bound graph according to an embodiment of the present invention;
Fig. 12 is a diagram illustrating a demonstration of an evaluation on a bound graph according to an embodiment of the present invention;
Fig. 13 is a diagram specifically illustrating a process for generating pathway-logic control value information used in a display program according to an embodiment of the present invention;
Fig. 14 is an enlarged diagram of Fig. 13;
Fig. 15 is an enlarged diagram of Fig. 13;
Fig. 16 is a diagram specifically illustrating a process for comparing pathway-logic control data used in a display program according to an embodiment of the present invention with experimental values (gene expression data);
Fig. 17 is an enlarged diagram of Fig. 16;
Fig. 18 is an enlarged diagram of Fig. 16;
Fig. 19 is an explanatory diagram illustrating how to obtain observed data according to an embodiment of the present invention;
Fig. 20 is a diagram specifically illustrating bound-graph drawing data according to an embodiment of the present invention;
Figs. 21A and 21B are diagrams specifically illustrating bound graphs according to an embodiment of the present invention;
Figs. 22A and 22B are diagrams specifically illustrating bound graphs according to an embodiment of the present invention;
Fig. 23 is a flowchart illustrating an operation of how to use a display program according to an embodiment of the present invention;
Fig. 24 is a pathway diagram according to the related art; and
Fig. 25 is a line graph showing observed values according to the related art.

### DESCRIPTION OF THE PREFERRED EMBODIMENTS

It is to be noted that the present invention may be implemented by various different forms and should not be construed only by the embodiments described below.

The embodiments will be described below in the context of a program. However, as is obvious to those skilled in the art, the present invention may also be implemented as a system or method usable on a computer. The present invention may be implemented by either a hardware embodiment or a software embodiment, or a software and hardware embodiment. The program can be recorded on any computer-readable medium, such as a hard disk, a compact disc read-only memory (CD-ROM), a digital versatile disc read-only memory (DVD-ROM), an optical storage device, or a magnetic storage device. The program can also be recorded in another computer via a network.

### 1. Overview of Display Program

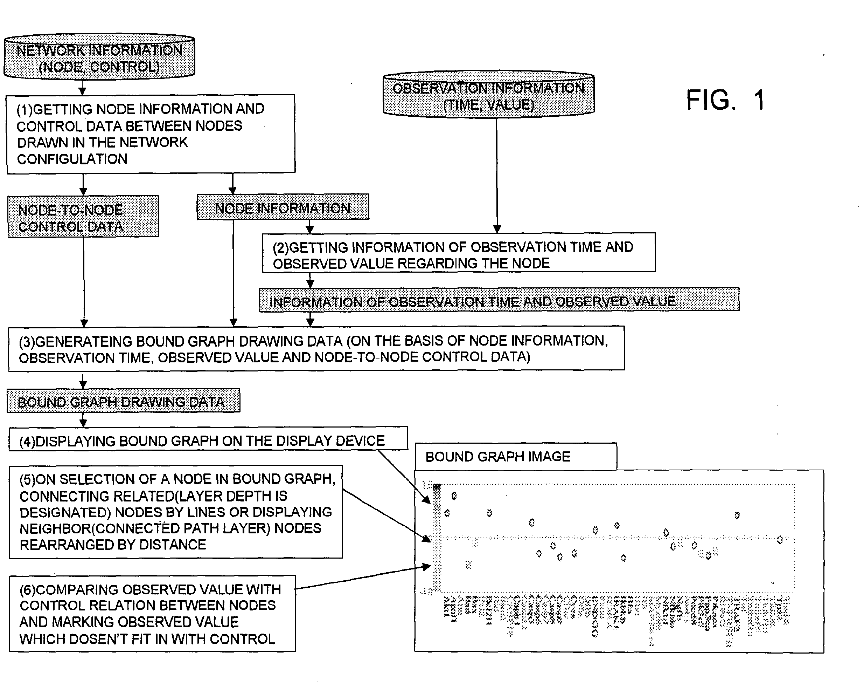

Fig. 1 is a schematic diagram illustrating a display program according to an embodiment of the present invention. The display program according to the embodiment causes a computer to execute a step of reading node information and control data that constitute network information from a first storage means (step (1) in Fig. 1) , a step of reading observation times and observed values associated with the read node information from a second storage means (step (2) in Fig. 1), a step of, on the basis of the read node information, observation times, and observed values, switching views for the respective observation times at predetermined intervals to display a two-dimensional representation relating to an observation time and having one axis representing the observed values and the other axis representing nodes (steps (3) and (4) in Fig. 1), a step of displaying a control relationship on the two-dimensional representation in response to a user specification (step (5) in Fig. 1), and a step of designating a node or nodes not fitting in with the displayed control relationship in the nodes associated with the displayed control relationship according to the control data and the observed values (step (6) in Fig. 1). Rearrangement of the displayed nodes in the step (5) in Fig. 1 is discussed below.

In the step (1) or (2) in Fig. 1, the storage means includes a storage device (such as a hard disk or a main memory) of a computer. In a broader sense, the storage means also includes a database. The first storage means and the second storage means may be the same. The step (1) or (2) may be triggered by an operation of a user, adding new observed data, specifying time, or the like. The new observed data may be added by automatically performing the steps described above on the additional observed data, or may be added only when observed data relating to a node specified by the user is added.

### 2. Hardware Configuration

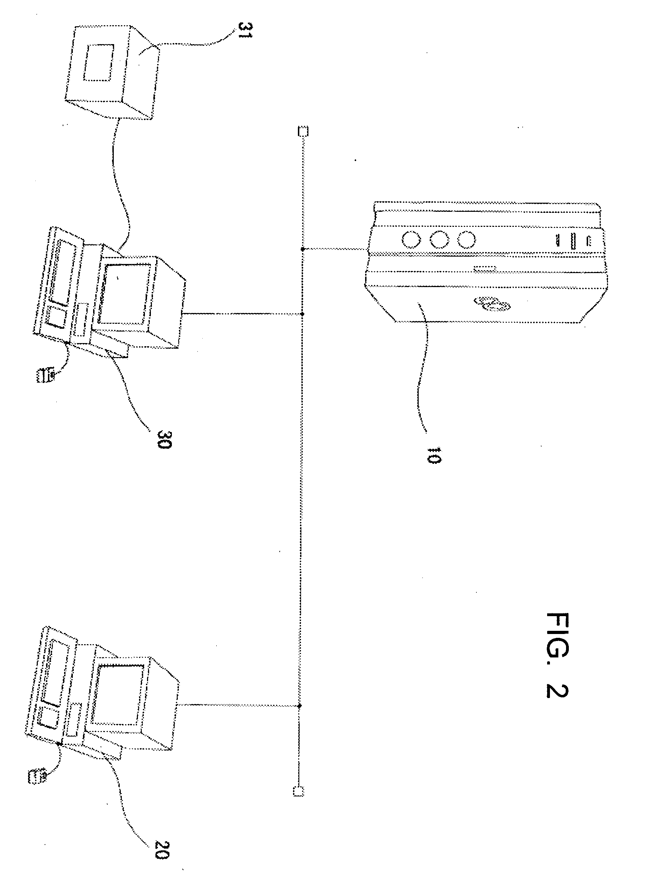

Fig. 2 is a configuration diagram of a system used in a display program according to an embodiment of the present invention. The system includes a mainframe computer 10 that constructs a database for recording various types of information, an analyzing computer 20, which is a personal computer adapted to execute the display program according to the present invention, and a detecting computer 30, which is a personal computer connected to a detector (scanner) 31 via a wired cable for converting an intensity of a fluorescent signal detected by the detector 31 into a numerical value and for determining the expression level of each gene. The expression level is an example of observed data. A pathway diagram is an example of network diagram.

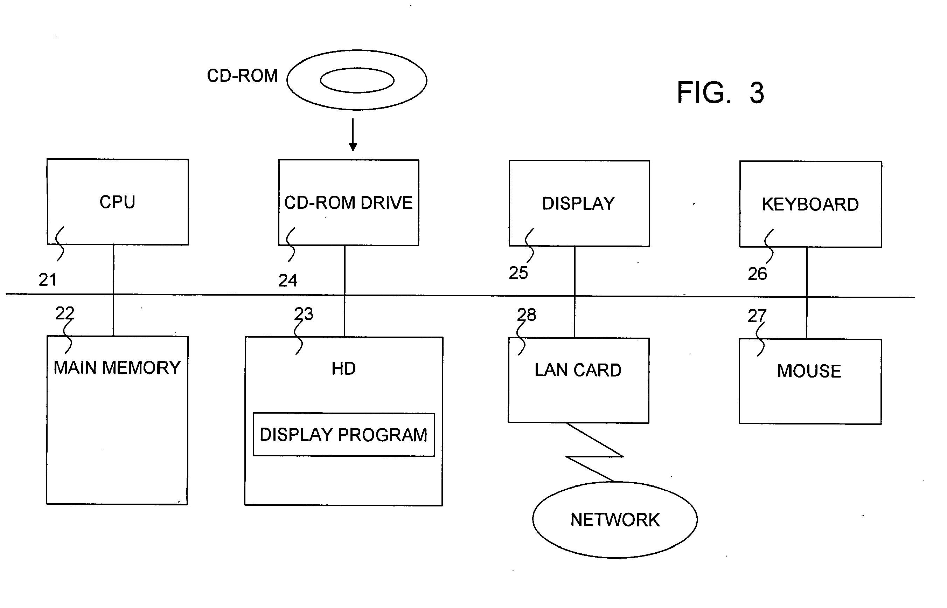

Fig. 3 illustrates an exemplary hardware configuration according to an embodiment. In the embodiment, a general personal computer with a hardware configuration including a central processing unit (CPU) 21, a main memory 22, a hard disk (HD) 23, a CD-ROM drive 24, a display 25, a keyboard 26, a mouse 27, and a local-area-network (LAN) card 28 is used as the analyzing computer 20. The mainframe computer 10 and the detecting computer 30 have a similar hardware configuration to that of the analyzing computer 20. The analyzing computer 20 and the detecting computer 30 may be personal computers. Desirably, the mainframe computer 10 is constructed of, for example, a rack-mounted server with a server rack including a plurality of CPUs, and includes a hard disk cabinet, a back-up cabinet, an uninterruptible power supply (UPS), a display mound, and a keyboard. However, the mainframe computer 10 can be implemented by a personal computer.

The database constructed in the mainframe computer 10 stores node information, network information, and observed data.

### 3. Bound Graph

### 3.1 Normal View Mode

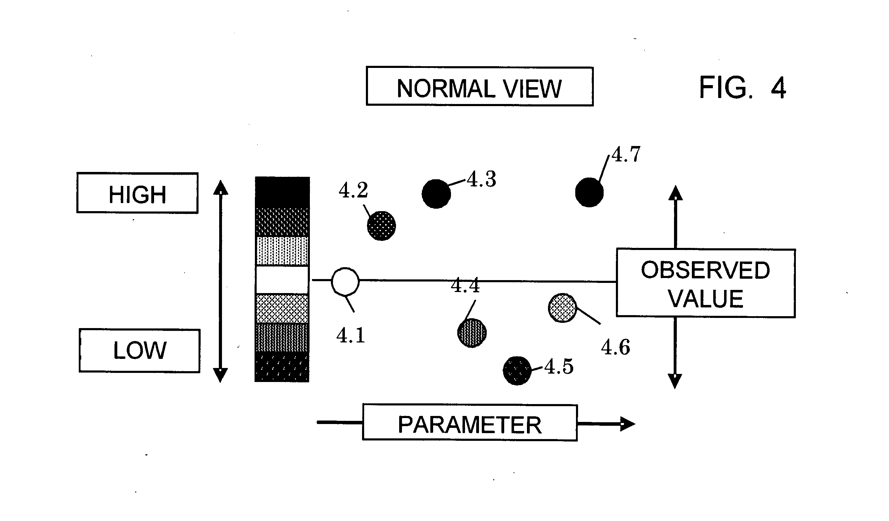

Fig. 4 illustrates an example bound graph in a normal view mode according to an embodiment of the present invention. In Fig. 4, a two-dimensional representation with the y-axis representing observed values and the x-axis representing genes (nodes) is illustrated. Measurement points of the nodes are shown in different colors from one observed value to another. For example, red components are assigned more than the remaining color components in the three primary colors to higher observed values, and green components are assigned more than the remaining color components are assigned to lower observed values. Fig. 4 illustrates measurement points (4.1, 4.2, 4.3, 4.4, 4.5, 4.6, and 4.7) representing the observed values of the gene expression levels at an observation time. In the example illustrated in Fig. 4, observed values of expression levels of seven genes are illustrated. In this embodiment, the observed values of the gene expression levels are determined by the detecting computer 30, and are stored in the mainframe computer 10. Each of the observed values has a data structure in which elements of the sequence include expression levels obtained at predetermined observation times. The observed values of the gene expression levels are plotted for each observation time, and the plots for the respective observation times are displayed in a switching manner at predetermined time intervals. By doing so, the measurement points of the nodes are displayed using animation effects as if moving up and down. Such a view, which resembles a view of a ball falling to the ground and bounding, is referred to a bound graph.

This allows the user to observe the variations of the observed values by using the movement of the measurement points in addition to coloring, which is easy-to-analyze for the user. The human eye is sensitive to the movement of the object more than the change in color of the object.

In a case where a plurality of observed data items are obtained with respect to a given node, such as a case where a plurality of experiments are made, a plurality of measurement points for the given node are displayed on the two-dimensional representation, thus allowing the user to examine the given node. For example, in a case where measurement points for a given node are spread across a wide range and a case where measurement points for a given node are concentrated in a narrow range, it is more desirable that the latter case be analyzed to obtain significant information.

### 3.2 Vector View Mode

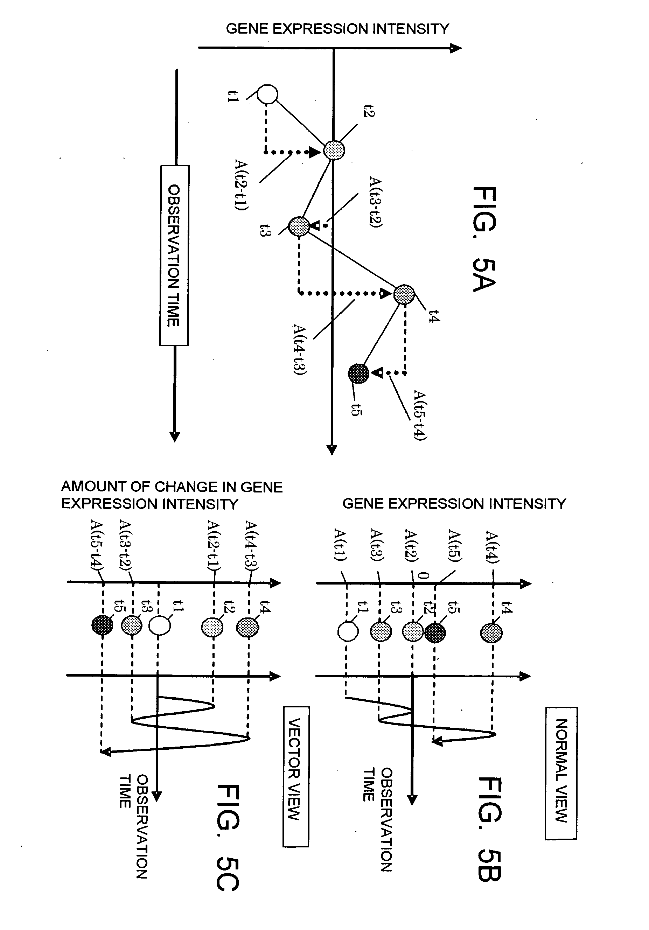

Figs. 5A to 5C illustrate an example bound graph in a vector view mode according to an embodiment of the present invention. In the vector view mode, changes in the gene expression intensity are plotted on a bound graph. In the vector view mode, therefore, the amount of change in the expression intensity (expression level) of a given gene between adjacent observation times is represented by a bound graph. This view mode enables the user to specify data having variations in the same direction as key data (node).

Fig. 5A is a graph relating to the same node, with the y-axis representing observed values (gene expression intensities) and the x-axis representing observation times (predetermined times at which the gene expression intensity of the given gene was observed). In Fig. 5A, the gene expression intensities at five measurement points, that is, measurement points t1, t2, t3, t4, and t5, are illustrated. An amount of change A(t2-t1) between the measurement points t1 and t2, an amount of change A(t3-t2) between the measurement points t2 and t3, an amount of change A(t4-t3) between the measurement points t3 and t4, and an amount of change A(t5-t4) between the measurement points t4 and t5 are' illustrated.

On the left side as viewed in Fig. 5B, the data string indicating the gene expression intensities illustrated in Fig. 5A is plotted on a bound graph in the normal view mode. Actually, the plots for the individual observation times are displayed in a switching manner at predetermined time intervals, and the measurement points are displayed using animation effects as if moving up and down as the observation times are changed. On the right side as viewed in Fig. 5B, an arrowed curve representing the trajectory of the measurement points illustrated in the left portion as viewed in Fig. 5B is illustrated in a plot with the y-axis representing the observed values and the x-axis representing the observation times. In the animation representation, the measurement points may be shown so that the current observed values are immediately changed to the next observed values when shifting to the next observation time, or may be shown so that the measurement points move to the next observed values.

Fig. 5C is a plot of the data string indicating the gene expression intensities illustrated in Fig. 5A on a bound graph in the vector view mode. The observation times of each node illustrated in Fig. 5A are referred to, starting from the origin, as time 1 (the first day, corresponding to the measurement point t1 in Fig. 5A), time 2 (the third day, corresponding to the measurement point t2 in Fig. 5A), time 3 (the seventh day, corresponding to the measurement point t3 in Fig. 5A), time 4 (the 14th day, corresponding to the measurement point t4 in Fig. 5A), and time 5 (the 28th day, corresponding to the measurement point t5 in Fig. 5A). In Fig. 5C, the measurement point t1 is plotted at the amount of change 0 because the node at time 1 is the point of origin, the measurement point t2 is plotted at the amount of change A(t2-t1) between times 1 and 2, the measurement point t3 is plotted at the amount of change A(t3-t2) between times 2 and 3, the measurement point t4 is plotted at the amount of change A(t4-t3) between times 3 and 4, and the measurement point t5 is plotted at the amount of change A(t5-t4) between times 4 and 5.

### 3.3 View Mode based on Movement Distance from Initial Observation time

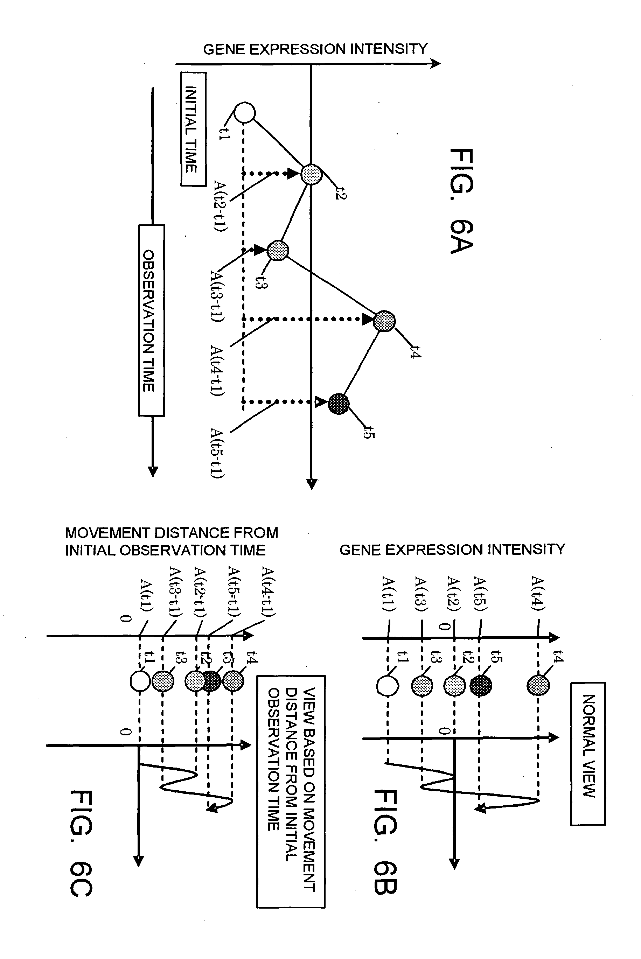

Figs. 6A to 6C illustrate an example bound graph in a view mode based on a movement distance from an initial observation time according to an embodiment of the present invention. In the view mode based on the movement distance from the initial observation time, the time corresponding to the initial observation time is set to 0, and the subsequent times are set to values determined by subtracting the observed value at the initial observation time from the observed values at the times, which are plotted on the y-axis. Figs. 6A and 6B correspond to Figs. 5A and 5B, respectively. Fig. 6C is a view obtained when the view of Fig. 6A is represented on the basis of the movement distance from the initial observation time. The node at time 2 is plotted at the value determined by subtracting the observed value of the node at time 1, which is the initial observation time, from the observed value of the node at time 2. The node at time 3 is plotted at the value determined by subtracting the observed value of the node at time 1, which is the initial observation time, from the observed value of the node at time 3. The nodes at times 4 and 5 are also plotted at the values determined by similar processing.

For example, in a graph of an experiment that requires a long time to change observed values, such as an experiment on drug administration, differences from the initial observation time are important. The view mode based on the movement distance from the initial observation time allows the user to easily compare the target node at the initial observation time with the target node at the current observation time, and is analytically important.

### 3.4 View Mode based on Distance between Two or More Parameters

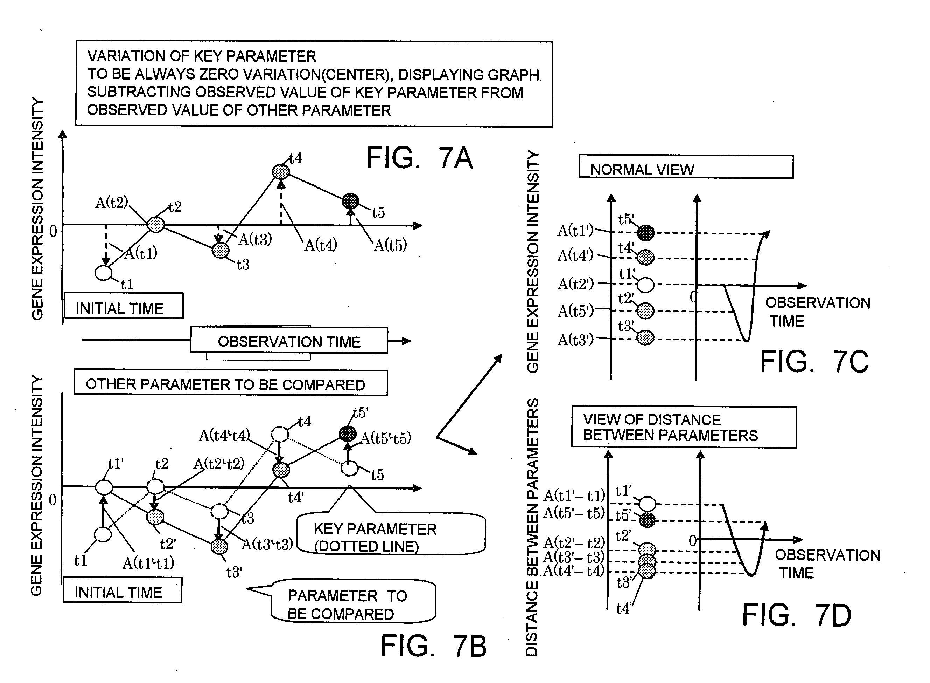

Figs. 7A to 7D illustrate an example bound graph in a view mode based on a distance between two or more parameters according to an embodiment of the present invention. In the view mode based on the distance between two or more parameters, a key node (reference parameter; key data) has always zero variation, and distances between the key node and other comparative nodes at each observation time are plotted on a graph. This allows the user to easily compare a key node with a target node. In the view mode based on the distance between parameters, the larger the up-and-down variation of the measurement point, the larger the difference in variation profile between the comparative node and the key node. Fig. 7A is a graph relating to the key node, with the y-axis representing observed values and the x-axis representing observation times. Fig. 7B is a graph relating to a target node, with the y-axis representing observed values and the x-axis representing observation times. In Fig. 7B, for the purpose of comparison, the graph illustrated in Fig. 7A is also illustrated, as indicated by a dotted line. Fig. 7C corresponds to Fig. 5B. Fig. 7D is a view obtained when the view of Fig. 7B is represented on the basis of the distance between the parameters. In the example illustrated in Fig. 7D, a measurement point t1' of the comparative node (target node) is plotted at a value A(t1'-t1) determined by subtracting an observed value A(t1) of the key node at time 1 from an observed value A(tl') of the target node at time 1. Measurement points t2', t3', t4', and t5' of the target node at times 2, 3, 4, and 5 are also plotted at values determined by similar processing.

The view mode based on the distance between two or more parameters allows examination of genes having the same movement as a reference gene.

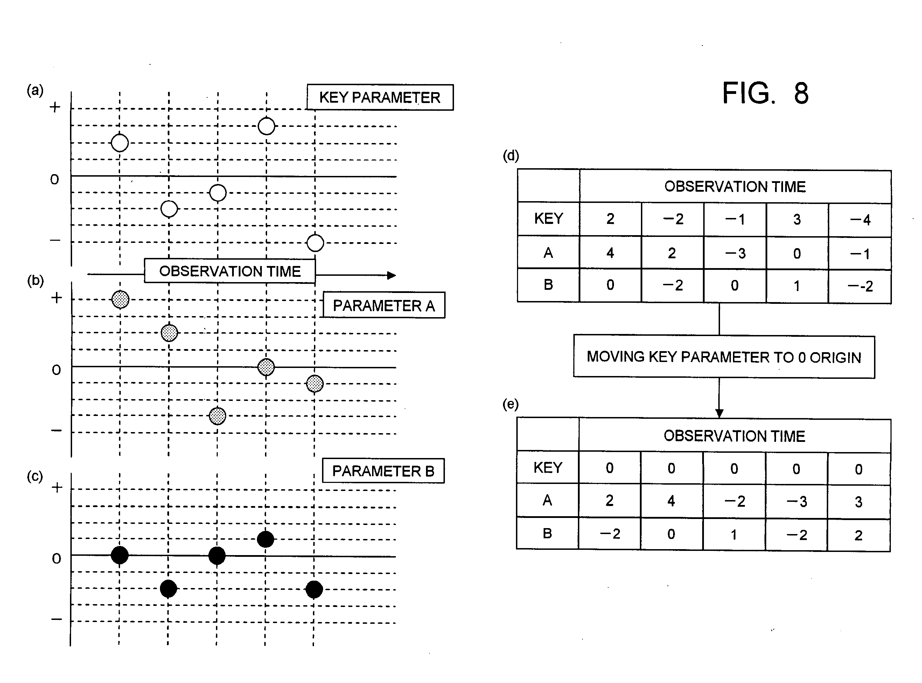

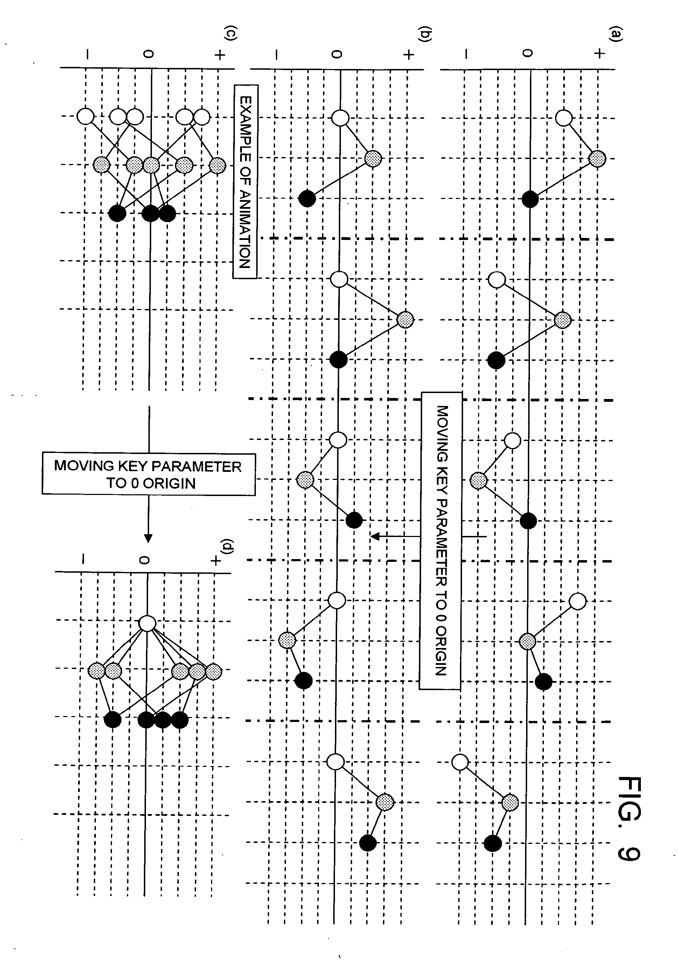

Figs. 8 and 9 specifically illustrate the view mode based on the distance between two or more parameters according to an embodiment. In Fig. 8, parts (a), (b), and (c) illustrate graphs relating to the key node, a node A, and a node B on the basis of a table illustrated in part (d), respectively, in which the y-axis represents observed values and the x-axis represents observation times. The table illustrated in part (d) of Fig. 8 is converted by setting the values of the key node to 0, thereby generating a table illustrated in part (e) of Fig. 8. In Fig. 9, a graph illustrated in part (a) is a graph in which the graphs illustrated in parts (a), (b), and (c) of Figs. 8 are plotted with respect to each time. In Fig. 9, a graph illustrated in part (b) is obtained on the basis of the graph illustrated in part (a) by using the key node as a reference. In other words, the graph illustrated in part (b) of Fig. 9 is a graph relating to the key node, the node A, and the node B on the basis of the table illustrated in part (b) of Fig. 8, in which the y-axis represents observed values and the x-axis represents observation times. In the graphs illustrated in parts (a) and (b) of Fig. 9, the times are separated by one-dot chain lines. In practice, however, as illustrated in parts (c) and (d) of Fig. 9, sets of the nodes at each time are represented with respect to a predetermined period of time.

### 3.5 Data-Type-Based Color-Coded Parameter View Mode

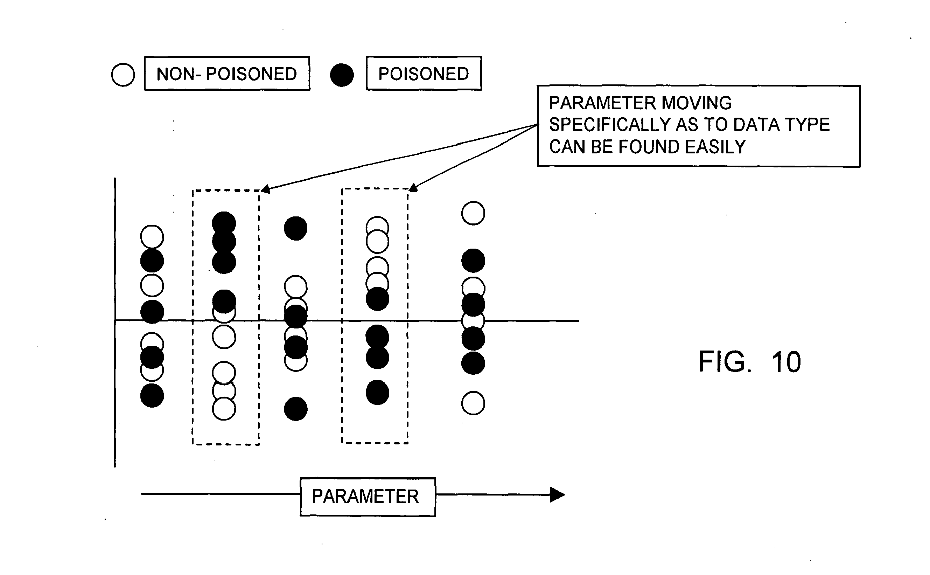

Fig. 10 illustrates a bound graph in a data-type-based color-coded parameter view mode according to an embodiment of the present invention. In the data-type-based color-coded parameter view mode, two or more experiments on the same node are represented by a plurality of graphs. The data-type-based color-coded parameter view mode allows the user to observe specific movement in data. In sections 3.1 to 3.4, the bound graphs have been discussed in the context of an experiment having one observed value at one time. In the view illustrated in Fig. 10, a plurality of experiments are simultaneously represented. The view illustrated in Fig. 10 is based on a result of performing two experiments a plurality of times. More specifically, experiments were made using, for example, four entities each to which a dose of poison was given and four entities each to which a dose of poison was not given, and experimental results were used. In the experimental results, the poisoned entities and the non-poisoned entities were biased at two nodes. It can be recognized that, for example, two genes were greatly affected by the toxic compound used herein.

### 4. Bound Graph and Network Control Relationship

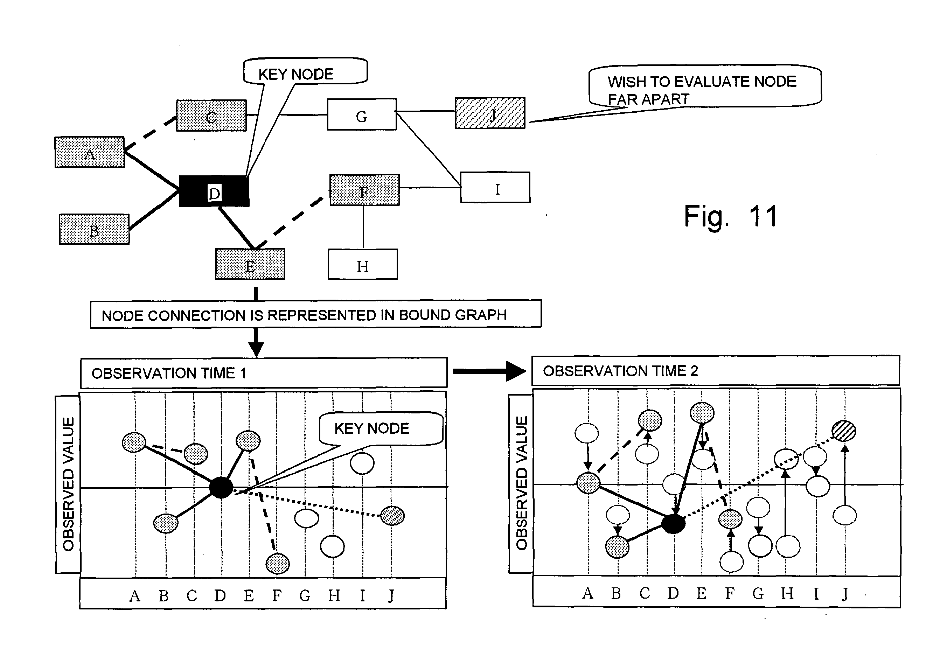

Fig. 11 illustrates a demonstration of a control relationship on a bound graph according to an embodiment of the present invention. Part (a) of Fig. 11 illustrates a control relationship between nodes A to J. Part (b) of Fig. 11 illustrates a bound graph for an observation time 1, with the y-axis representing observed values and the x-axis representing nodes. Part (c) of Fig. 11 illustrates a bound graph for an observation time 2, with the y-axis representing observed values and the x-axis representing nodes. The node D is a key node (target node), and control relationships are represented in two layers. Other than the node D and the nodes having the relationships, the node J is a node to be compared with the node D. In parts (b) and (c) of Fig. 11, the control relationship in the first layer is represented by a solid line, the control relationship in the second layer is indicated by a dashed line, and the control relationship between the key node and the node to be compared is represented by a dotted line.

In this manner, the control relationships are also displayed together with the bound graph, thus allowing the user to simultaneously view the variations of the nodes and the control relationships in parallel, and to understand the observed data and the control relationship between the desired nodes merely by viewing the two-dimensional representation, which helps the user perform the analysis more smoothly.

### 5. Network Control and Evaluation of Observed Values

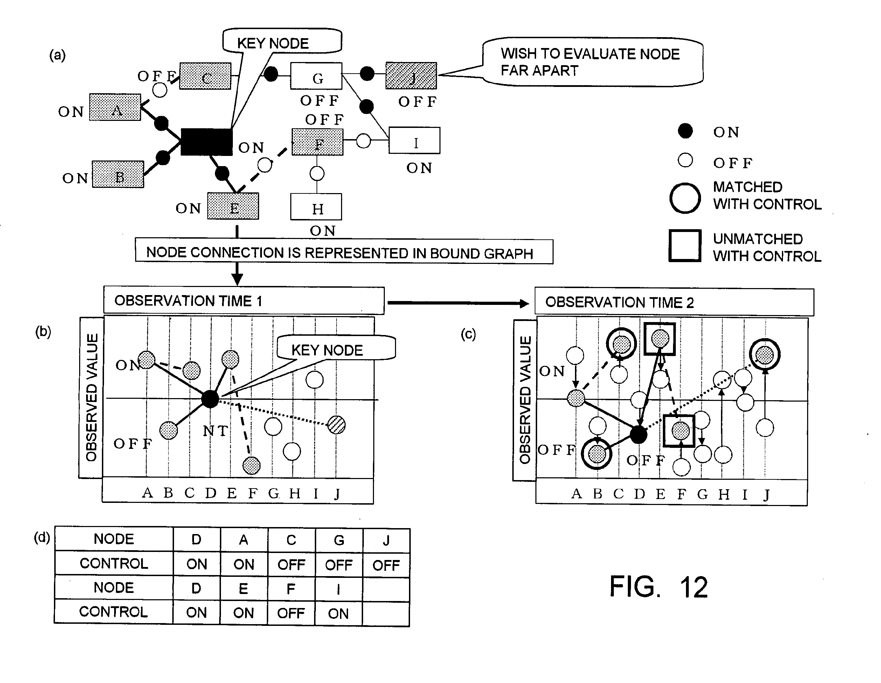

Fig. 12 illustrates a demonstration of an evaluation on a bound graph according to an embodiment of the present invention. An evaluation is performed as to whether or not network control data (e.g., ON or OFF) and an actual observed value match each other, and evaluation results are represented by a bound graph. In Fig. 12, a node whose control relationship and observed value match each other is surrounded by a circle, and a node whose control relationship and observed value do not match each other is surrounded by a square. A table illustrated in part (d) of Fig. 12 shows exemplary network control relationships (an ON state (indicated by a black circle) and an OFF state (indicated by a white circle)). For example, when the node D is in the ON state, the node A is turned on. The ON or OFF state of each node is controlled depending on the relationship between the observed value of this node and a threshold value. For example, if the gene expression intensity is a predetermined value or higher, the ON state is entered; if the gene expression intensity is lower than the predetermined value, the OFF state is entered.

Parts (b) and (c) of Fig. 12 illustrate bound graphs for the observation times 1 and 2, respectively, with the y-axis representing the observed values and the x-axis representing the nodes. The bound graph illustrated in part (c) of Fig. 12 illustrates an evaluation as to whether or not the variations of the observed values fit in with the control relationships when the observation time 1 is shifted to the observation time 2.

Along with such a bound graph, both the control relationships and the evaluation as to whether or not the variations fit in with the control relationships are represented. This allows the user to understand the control relationship between the nodes to rapidly and satisfactorily perform an analysis without determining whether or not each of the nodes is a node whose observed value fits in with the control relationship. Since the user does not need to perform the determination as to the fitness for the control relationship, the analysis can rapidly be performed and the ability of the user that is not used for the determination is assigned to the analysis.

### 6. Data Structure and Process Flow

### 6.1 Data Structure

### 6.1.1 Network Information

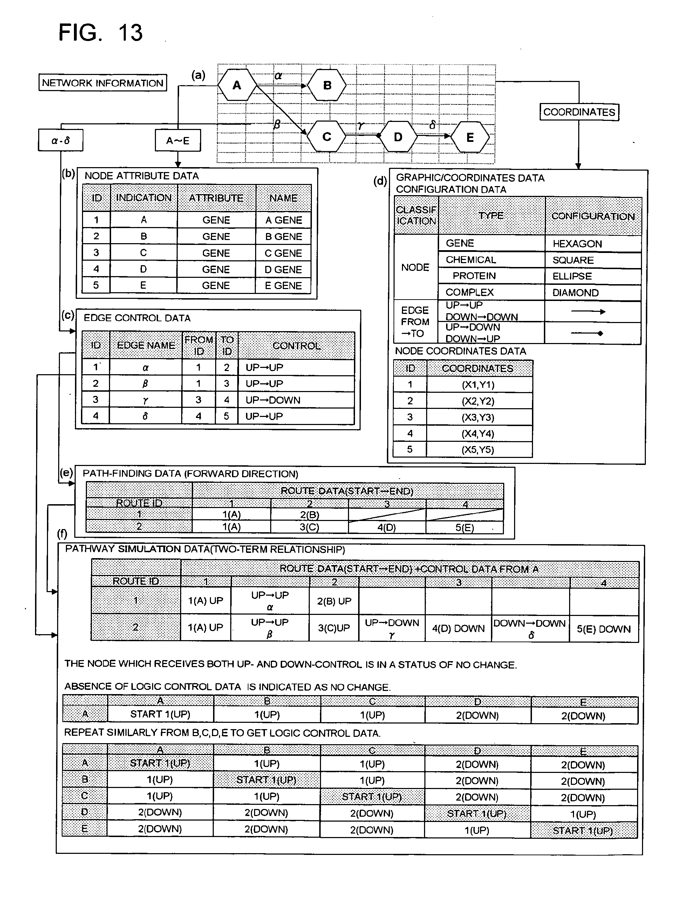

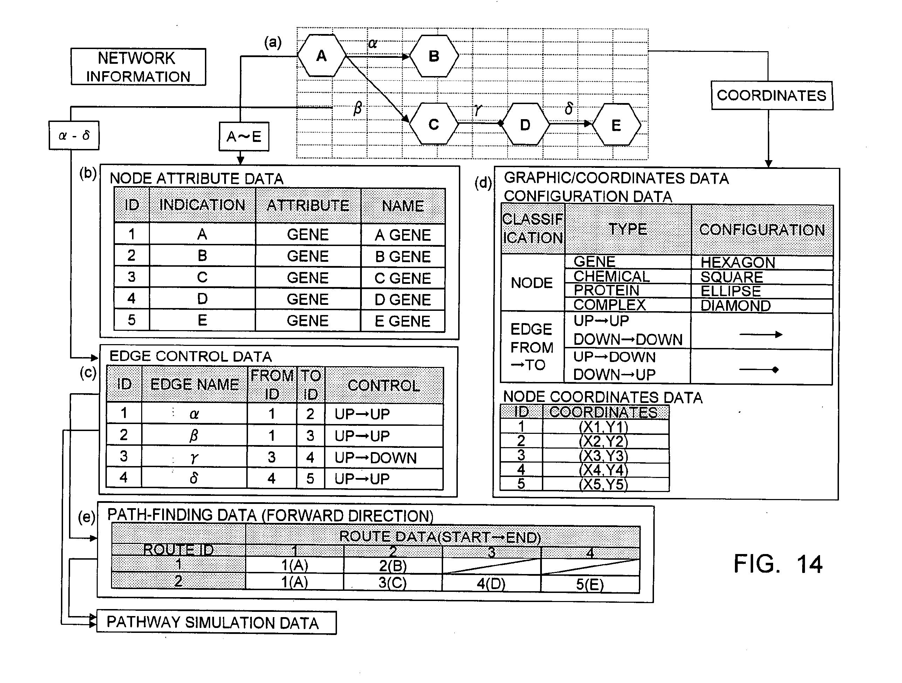

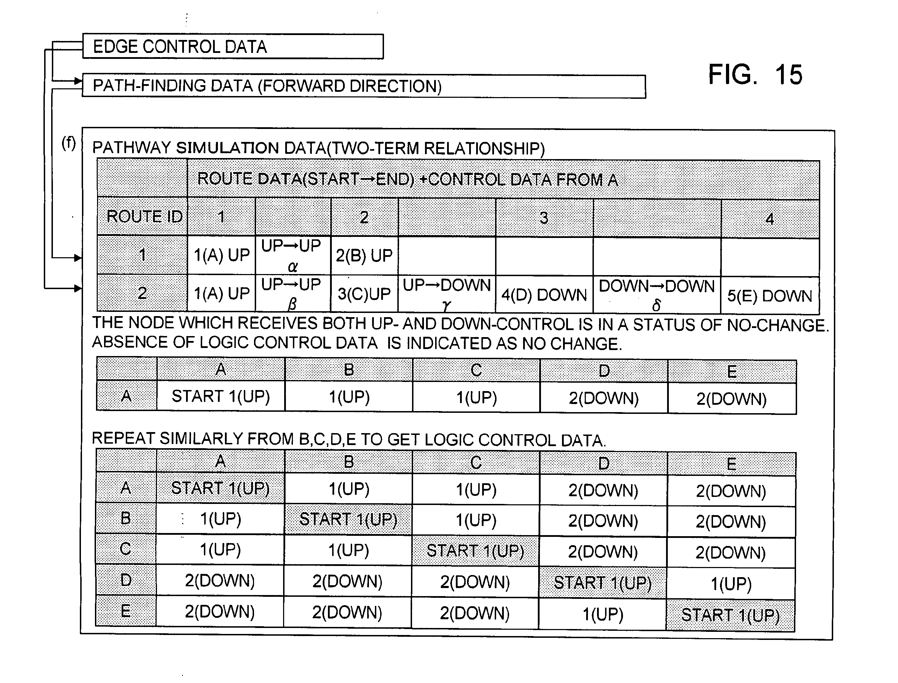

Fig. 13 is a diagram specifically illustrating a process for generating pathway-logic control value information used in a display program according to an embodiment of the present invention. Figs. 14 and 15 are enlarged diagrams of Fig. 13 for clarity. Elements of node attribute information constituting a pathway include an ID, an indication, an attribute, and a name. Elements of edge control data constituting the pathway include an ID, an edge name, a From-ID, a To-ID, and control. Graphic/coordinates data constituting the pathway includes configuration data and node coordinates data. Elements of the configuration data include a classification, a type, and a configuration. Elements of the node coordinates data include an ID and coordinates.

The CPU 21 can use various route-finding algorithms to determine path search information from the edge control data. In Fig. 13, for convenience of illustration, the pathway is simple, and only two routes exist. Thus, the routes are readily found. If a large number of nodes and branches exist, an infinite number of routes can be found. In such a case, it is desirable that the longest route be found (route search process). By doing so, the number of routes in the path search information can be reduced. The longest route can be found by various methods. In an exemplary method of simply finding a traceable route and, when experiencing a dead end, going back to the branch portion to search for another route, after finding all routes, the longest route is selected from the found routes (for example, the longest route is determined on the basis of the number of nodes routed). With respect to each of the routes in the path search information that is determined by the CPU 21 using the node A as the starting point, edges corresponding to node-to-node lines and controls are determined from the edge control data. Since the node A is set as the starting point, the routes of route ID 1 and route ID 2 are represented by the top and middle tables illustrated in part (f) of Fig. 13. Similarly to the node A, when each of the nodes B, C, D, and E is set as the starting point, logic control data shown in the bottom table illustrated in part (f) of Fig. 13 is determined (virtual protein and gene status determination process). The logic control data indicates the status of other genes when the status of a given gene is determined. As can be seen from the logic control data, for example, when a gene A is active, genes B and C are active and genes D and E are inactive. Since each gene has two statuses, a binary relationship can be set. As can also be seen, when the gene A is inactive, the genes B and C are inactive and the genes D and E are active. Such logic control data can be determined from pathways. It is therefore desirable that logic control data be obtained in advance in the database of the mainframe computer 10.

### 6.1.2 Comparison between Observed data and Network Information

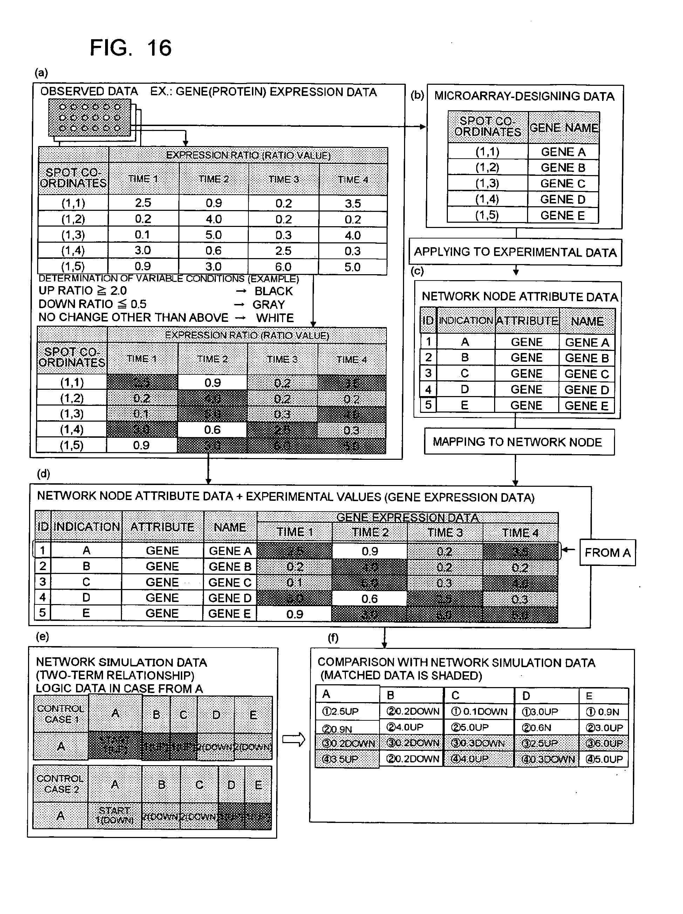

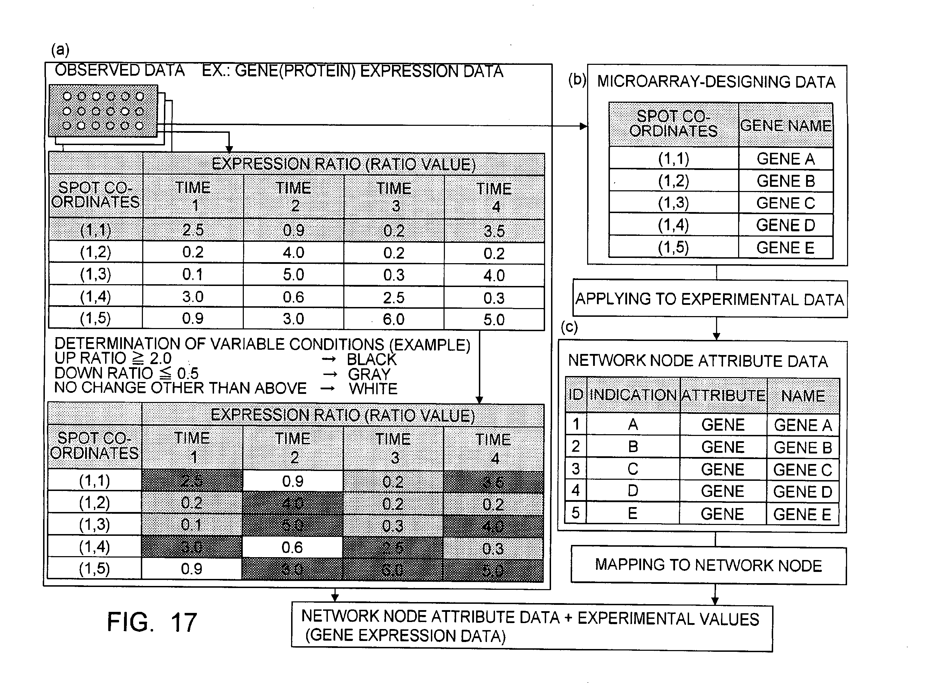

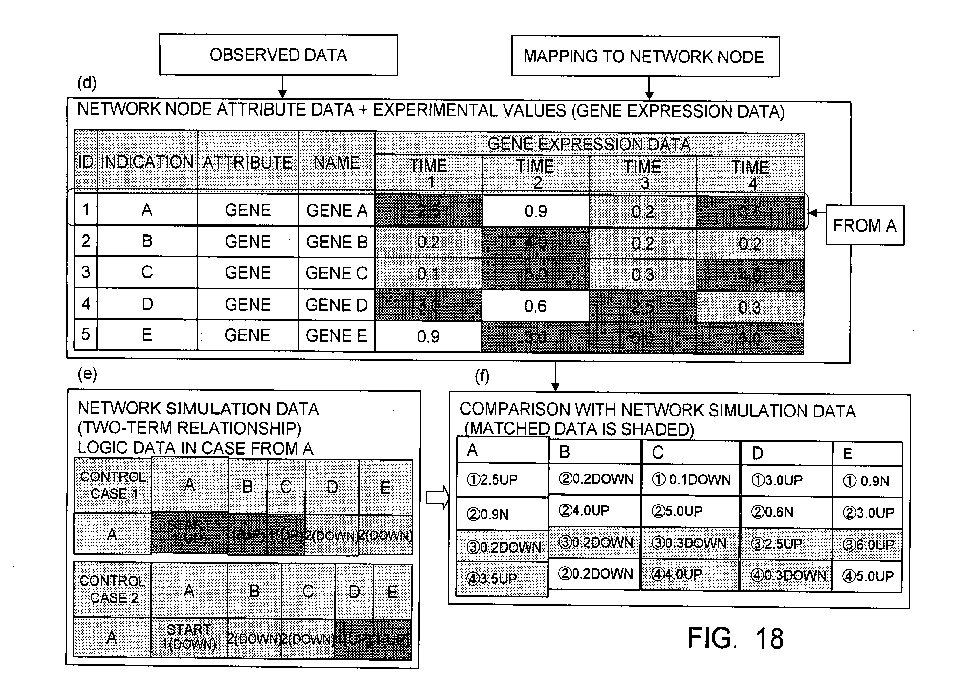

Fig. 16 is a diagram specifically illustrating a process for comparing pathway-logic control data used in a display program according to an embodiment of the present invention with experimental values (gene expression data). Figs. 17 and 18 are enlarged diagrams of Fig. 16 for clarity. The gene expression data includes spot coordinates, an expression ratio at time 1, an expression ratio at time 2, an expression ratio at time 3, and an expression ratio at time 4. This is merely an example, and the gene expression data may include gene expression data at more times. The bottom table in illustrated in part (a) of Fig. 16 is color-coded, for convenience of illustration, on the basis of the determination of the variation conditions. Elements of microarray design information shown in part (b) of Fig. 16 include spot coordinates and a gene name. The CPU 21 combines the gene expression data and the node attribute information according to the microarray design information to configure a table illustrated in part (d) of Fig. 16. Information shown in part (e) of Fig. 16 corresponds to the path simulation information shown in part (f) of Fig. 13. When the gene A is the key gene and time 1 is the current time, expression ratios of the genes B, C, D, and E at time 1 and the following times are constructed on the basis of the expression ratio of the gene A at time 1 so as to meet the path simulation information requirements (construction process). More precisely, no combination of a controlled gene and a controlling gene is generated, wherein the time of the controlled gene precedes the time of the controlling gene. This is because a controlled gene is controlled by a controlling gene to determine the gene status, and the time of the controlled gene does not generally precede the time of the controlling gene although the time of the controlling gene and the time of the controlled gene would be identical. If doing so, the above-described combination which is not generally generated, i.e.., a combination of a controlled gene and a controlling gene, wherein the time of the controlled gene precedes the time of the controlling gene, is generated.

Expression ratios of the key gene A at times 2, 3, and 4 are also constructed by similar processing. Thus, the columns A to E in the table illustrated in part (f) of Fig. 16 are determined.

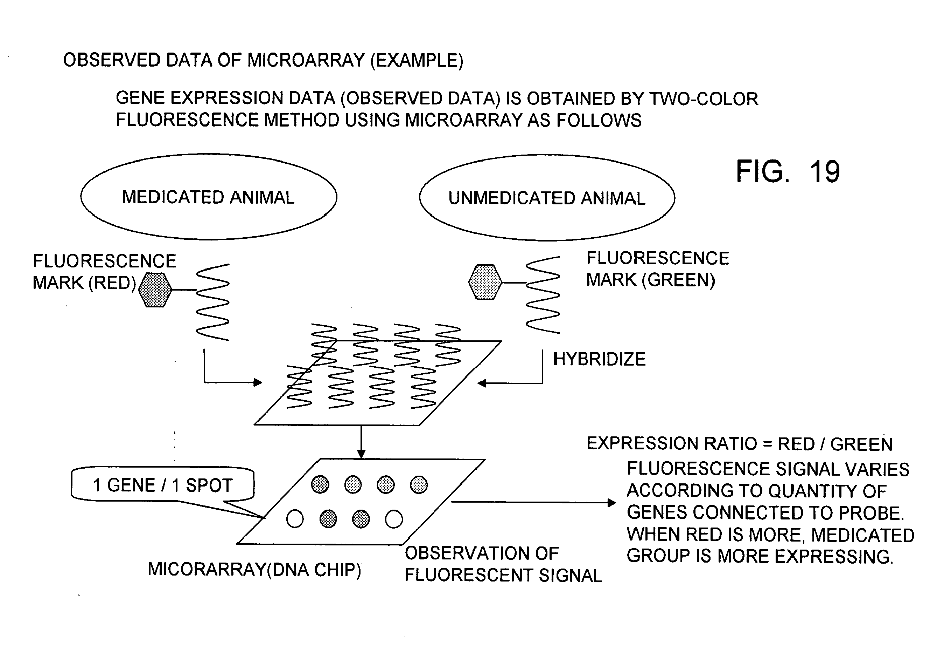

The expression data can be obtained using, for example, a gene expression analysis method using known microarray technology. In general, first, a probe DNA is prepared and is spotted onto a glass slide. Then, a target DNA is prepared and is conjugated with a fluorescent dye. Then, hybridization and washing are carried out, and a fluorescent signal is detected by the detector 31 and is digitized by the computer 30 to obtain expression data. The resulting gene expression data is stored in the database of the mainframe computer 10. More specifically, a procedure illustrated in Fig. 19 is performed. Fig. 19 is an explanatory diagram illustrating how to obtain observed data according to an embodiment of the present invention.

A pathway, which is genetic interaction information, can be obtained by collecting pathway fragments disclosed in papers. Some famous databases (such as EcoCyc, MetaCyc, KEGG, TRANSPATH, and CSNDB) are accessible via the Internet, and pathways can also be obtained from such famous databases. The pathways are stored in the database of the mainframe computer 10.

### 6.1.3 Bound Graph drawing data

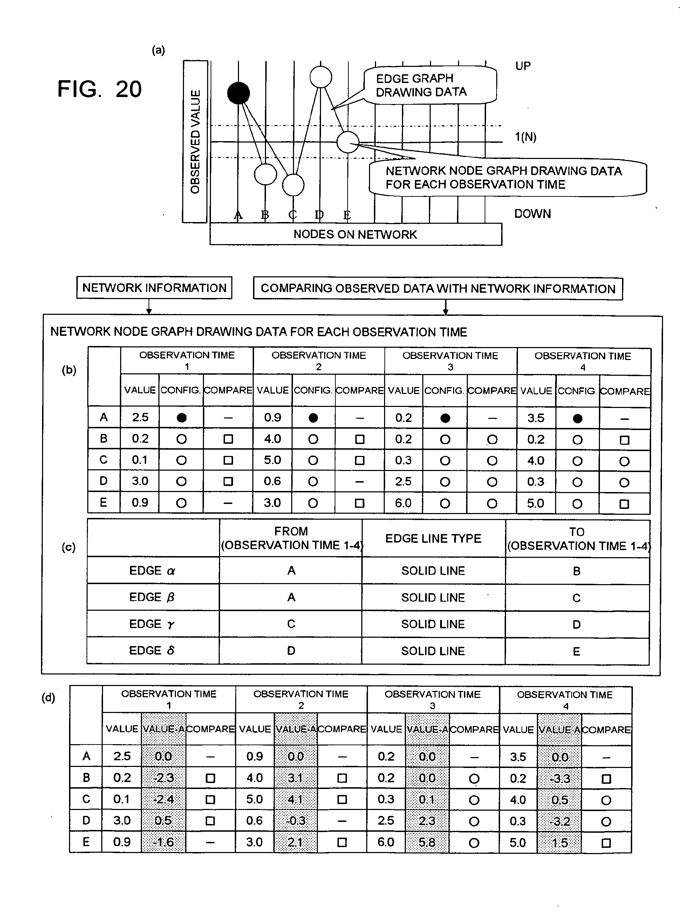

Fig. 20 is a diagram specifically illustrating bound-graph drawing data according to an embodiment of the present invention. The bound-graph drawing data is generated on the basis of network information and observed data. In network node graph information shown in part (b) of Fig. 20, a node A, which is the key node, has a black circular configuration, and other nodes have a white circular configuration. In the network node graph information, comparison represented by a white circle indicates that a match is found between each node and the corresponding network simulation value shown in part (f) of Fig. 16, comparison represented by a square indicates that no match is found, and comparison represented by a hyphen (-) indicates the key node itself or a neutral state.

### 6.2 Data Processing

### 6.2.1 Normal View Mode

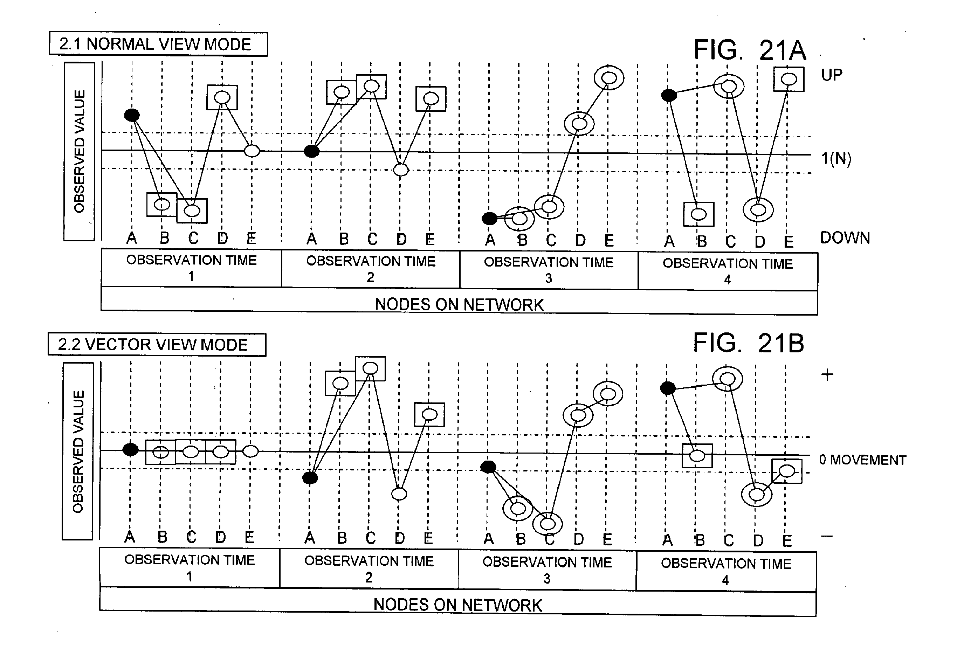

Figs. 21A and 21B are diagrams specifically illustrating bound graphs according to an embodiment of the present invention. The bound graphs illustrated in Figs. 21A and 21B are based on the bound-graph drawing data shown in Fig. 20. Fig. 21A illustrates the normal view mode.

### 6.2.2 Vector View Mode

Fig. 21B illustrates the vector view mode.

### 6.2.3 View Mode based on Movement Distance from Initial Observation time

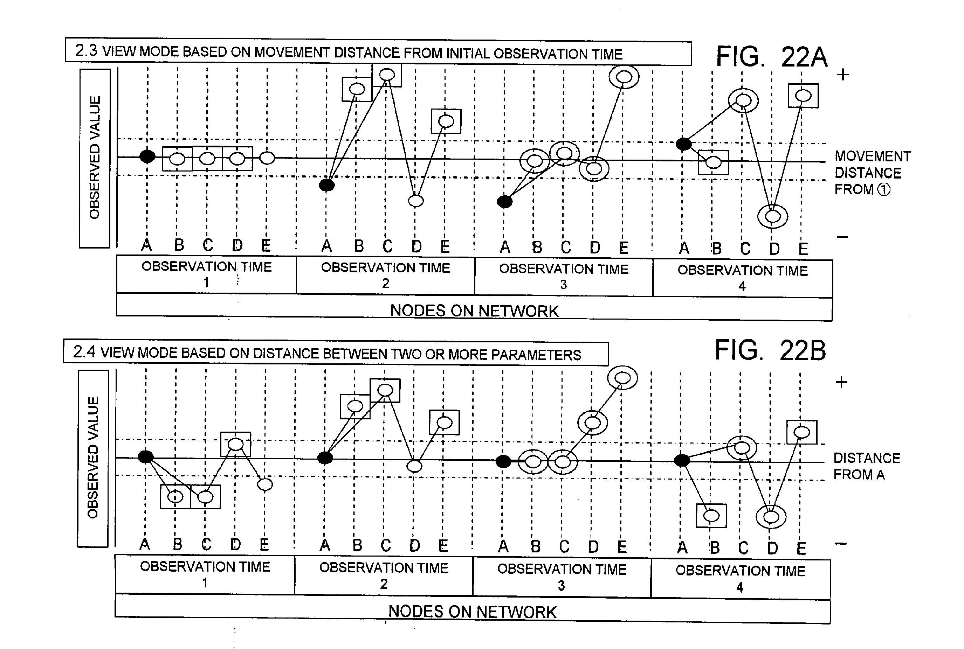

Figs. 22A and 22B are diagrams specifically illustrating bound graphs according to an embodiment of the present invention. The bound graphs illustrated in Figs. 22A and 22B are based on the bound-graph drawing data shown in Fig. 20. Fig. 22A illustrates the view mode based on the movement distance from the initial observation time.

### 6.2.4 View Mode based on Distance between Two or More Parameters

Fig. 22B illustrates the view mode based on the distance between two or more parameters. The view illustrated in part (a) of Fig. 20 shows a computation result when the node A is used as a reference for comparison. The graph illustrated in Fig. 22B is based on the determined values.

### 7. Operation

### 7.1 Basic Operation

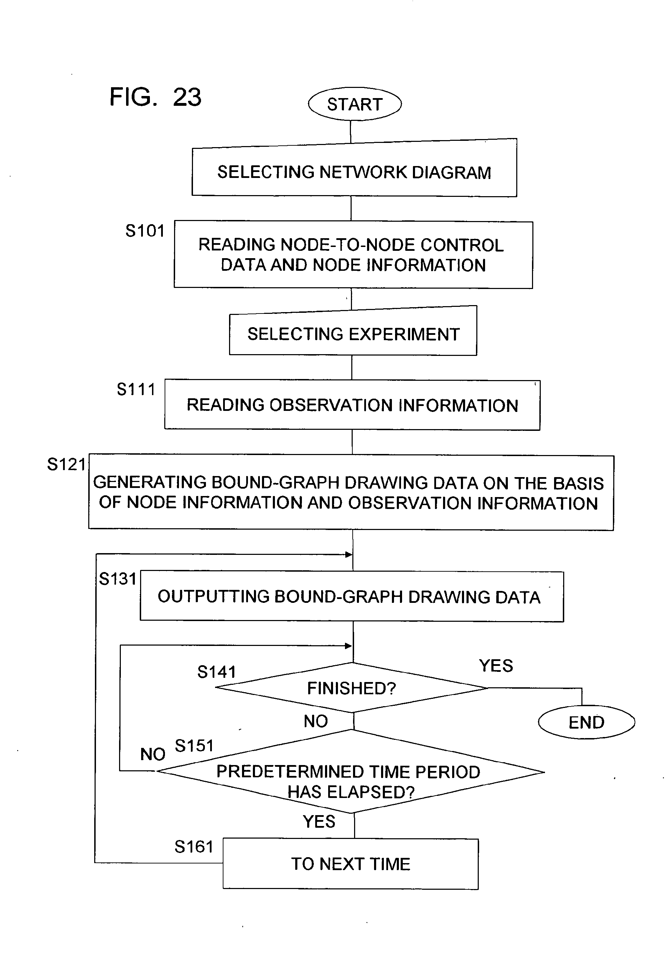

Fig. 23 is a flowchart illustrating an operation of how to use a display program according to an embodiment of the present invention.

A user selects a network diagram using an operation field (e.g., a switch, a pull-down menu, a tool bar, or a menu bar) displayed on the display 25. The CPU 21 reads node-to-node control data and node information of the selected network diagram from the database of the mainframe computer 10 (step 101). The user selects an experiment using the operation field displayed on the display 25. The CPU 21 reads the observed data of the selected experiment from the database of the mainframe computer 10 (step 111). The CPU 21 generates bound-graph drawing data on the basis of the read node information and observed data (step 121). The CPU 21 outputs the generated bound-graph drawing data to the display 25 (step 131). The CPU 21 determines whether or not the operation is finished (step 141). If the CPU 21 determines that the operation is finished, the flow ends. If the CPU 21 determines that the operation is not finished, the CPU 21 determines whether or not a predetermined period of time has elapsed (step 151). If the CPU 21 determines that the predetermined period of time has not elapsed, the flow returns to step 141. If the CPU 21 determines that the predetermined period of time has elapsed, the program shifts to the processing for the next time (step 161), and the flow returns to step 131.

The processing of steps 131, 151, and 161 allows the graph views to be changed to display an animation.

A bound graph is normally displayed in the normal view mode. In response to a selection by the user using the operation field, the view mode can be changed to the vector view mode, the view mode based on the movement distance from the initial observation time, or the view mode based on the distance between two or more parameters.

### 7.2 Demonstration of Control Relationship between Nodes

When the user selects nodes, the CPU 21 performs the processes illustrated in Figs. 14 and 16 to generate the bound-graph drawing data shown in Fig. 20 from the read network information and observed data relating to the selected nodes. Thus, a control relationship between the selected nodes is displayed on the display 25 in step 131, and a mark indicating a node fitting in with the control relationship and a mark indicating a node not fitting in with the control relationship are represented by a node-by-node basis.

The user may select nodes by specifying a key node and a layer (e.g., the node D and the second layer), specifying a key node and an endpoint node (e.g., the node A and the node D), selecting all nodes for which control relationships are to be shown (e.g., the nodes A, B, C, and D), or the like.

### 7.3 Rearrangement of Nodes on Graph

The user may also instruct rearrangement of the nodes. For example, in the bound graph illustrated in Fig. 1, nodes are alphabetically arranged (genes with similar names may be correlated from the derivations of the names). The nodes may be manually rearranged by the user specifying the positions of the nodes, or may be rearranged according to the layer from the key node, as previously discussed in section 7.2. Alternatively, the observed data may be subjected to cluster analysis to rearrange the nodes on a cluster-by-cluster basis. In the cluster analysis, for example, the nodes whose observed values have the same amount of change over the times are grouped into the same cluster. More specifically, when a node A has an observed value of 4.0 (at time 1) and an observed value of 6.0 (at time 2), a node B has an observed value of 4.0 (at time 1) and an observed value of 2.2 (at time 2), a node C has an observed value of 2.0 (at time 1) and an observed value of 0.1 (at time 2), and a node D has an observed value of 2.0 (at time 1) and an observed value of 4.0 (at time 2), the nodes A and D are grouped into the same cluster, and the nodes B and C are grouped into the same cluster. The nodes A, B, C, and D arranged in this order are thus rearranged into the nodes A, D, B, and C in this order.

The above-described operation is merely an example, and a variety of modifications of the operation can be conceived by those skilled in the art on the basis of the previous description in sections 1 to 6.

### 8. Advantages of the Embodiment

According to an embodiment of the present invention, therefore, observed data can be used to display a bound graph using animation effects, and a plurality of views can be selectively used depending on the type of the observed data, thus allowing the user to perform an appropriate analysis in accordance with the observed data. Along with the bound graph, a control relationship between selected nodes is also represented, as needed. The user does not need to switch between a graph representing the observed data and a network diagram. Moreover, since the CPU determines whether or not the observed data of the node fits in with the control relationship and displays the result, labor is not required to determine whether or not the observed data fits in with the control relationship, thereby rapidly and satisfactorily performing the analysis.

While the present invention has been described with reference to embodiments thereof, the technical scope of the present invention is not limited to the features disclosed in the embodiments, and a variety of modifications or improvements may be made to the embodiments. All of the features described hereinabove are not essential to the present invention, and both combinations and sub-combinations of these features may also fall within the present invention. Such modifications, improvements, combinations or sub-combinations also fall within the technical scope of the present invention. This is obvious in view of the appended claims and the summary of the invention.

## Claims

1. Program storage medium readable by a computer, tangibly embodying a program of instructions executable by the computer to perform a method for displaying two-dimensional representation relating to an object, said method comprising:
a reading step of reading an observation information of the object from a storage means, said observation information including an observation time and an observed value, said object being observed at the observation time and being associated with the observed value, said observation time and said observed value being stored in the storage means beforehand;
a generating step of generating drawing-data of the object on a basis of the observation time and the observed value; and
a displaying step of displaying the two-dimensional representations corresponding to a plurality of the observation times in turn on a display device, said two-dimensional representation being generated on a basis of the drawing-data, said two-dimensional representation having a first coordinate axis for representing an identifier of the object and a second coordinate axis for representing the observed value of the object.

2. The program storage medium of claim 1, wherein
said reading step comprises a sub-step of reading control data from a second storage means, said control data including control relationship between the objects and being stored in the second storage means beforehand,
said generating step is performed on a basis of the control data to generate the drawing-data, and
said displaying step comprises a sub-step of displaying an representation of the control relationship between the objects relating to a designated object in the two-dimensional representation on a basis of the control data, said designated object being designated by a user.

3. The program storage medium of claim 2, wherein
said displaying step comprises a sub-step of displaying an representation of a matched object distinctively, said matched object fitting in with the control relationship between the objects relating to the designated object.

4. The program storage medium of claim 1, 2 or 3, wherein
said two-dimensional representation has the second coordinate axis for representing a difference between a current observed value at a current observation time and a previous observed value at a previous observation time of the same object instead of the observed value of the object.

5. The program storage medium of claim 1, 2 or 3, wherein
said two-dimensional representation has the second coordinate axis for representing a difference between a current observed value at a current observation time and an initial observed value at an initial observation time of the same object instead of the observed value of the object.

6. The program storage medium of claim 1, 2 or 3, wherein
said two-dimensional representation has the second coordinate axis for representing a difference between an observed value of the object and an observed value of a key object instead of the observed value of the object, said key object being a predetermined object.

7. The program storage medium of claim 1, 2 or 3, wherein
said two-dimensional representation has a plurality of the observed values at the same observation time of the object.

8. The program storage medium of claim 1, wherein
arrangement of said identifiers on the first coordinate axis is changed according to an instruction of a user.

9. The program storage medium of claim 1, wherein
said object is a gene or protein, and
said observed value is relating to expression data of the gene or the protein.

10. Apparatus for displaying two-dimensional representation relating to an object, comprising:
a reading means for reading an observation information of the object from a storage means, said observation information including an observation time and an observed value, said object being observed at the observation time and being associated with the observed value, said observation time and said observed value being stored in the storage means beforehand;
a generating means for generating drawing-data of the object on a basis of the observation time and the observed value; and
a displaying means for displaying the two-dimensional representations corresponding to a plurality of the observation times in turn on a display device, said two-dimensional representation being generated on a basis of the drawing-data, said two-dimensional representation having a first coordinate axis for representing an identifier of the object and a second coordinate axis for representing the observed value of the object.

11. Method for displaying two-dimensional representation relating to an object, said method being performed by a computer, comprising:
a reading step of reading an observation information of the object from a storage means, said observation information including an observation time and an observed value, said object being observed at the observation time and being associated with the observed value, said observation time and said observed value being stored in the storage means beforehand;
a generating step of generating drawing-data of the object on a basis of the observation time and the observed value; and
a displaying step of displaying the two-dimensional representations corresponding to a plurality of the observation times in turn on a display device, said two-dimensional representation being generated on a basis of the drawing-data, said two-dimensional representation having a first coordinate axis for representing an identifier of the object and a second coordinate axis for representing the observed value of the object.
